# EUROPEAN PATENT APPLICATION

(11) **EP 4 674 403 A1**
(43) Date of publication of application: **07.01.2026**
(21) Application number: 23931856.1
(22) Date of filing: 21.12.2023
(51) Int. Cl.: A61K 8/25, A61K 8/37, A61K 8/34, A61K 8/891, A61K 8/73, A61K 8/81, A61K 8/88, A61Q 17/04, A61Q 15/00

(54) **ANTI-BACTERIAL AND SUNSCREEN SOLID-BLOCKY POWDER COMPOSITION HAVING ORNAMENT COMPONENT AND PREPARATION METHOD THEREFOR**

(30) Priority: 06.04.2023 CN 202310354832
(71) Applicant: Cosbe Laboratory Inc., Shantou, Guangdong 515000 (CN)
(72) Inventor: OR, Huen, Hong Kong 999077 (CN); ZHENG, Songwu, Shantou, Guangdong 515000 (CN); ZHANG, Liepeng, Shantou, Guangdong 515000 (CN); HUANG, Xiazhi, Shantou, Guangdong 515000 (CN); ZHUANG, Shanzhao, Shantou, Guangdong 515000 (CN)
(74) Representative: Chung, Hoi Kan
(86) International application number: PCT/CN2023/140697
(87) International publication number: WO 2024/207811

(57) **Abstract**

An antibacterial and sun-protective powdered solid compact composition with decorative elements, consisting of the following raw materials in percentage by weight: 15% to 25% of aluminum starch octenylsuccinate and/or calcium starch octenylsuccinate; 45% to 65% of a carrier powder; 1% to 10% of an ultraviolet protective agent; 0.1% to 0.5% of an antibacterial agent; 1% to 5% of a non-expandable polymer powder; 5% to 15% of an viscous fluid skin-feel modifier; 0.1% to 7.5% of a plant extract, wherein the solid raw materials have a particle size of 5-50 µm, and the raw materials are thoroughly mixed and compressed to form a powdered solid compact composition with a density of 8-9 g/cm³. The manufacturing process comprising pre-mixing, blending, pulverizing, standing, and compacting under appropriate pressure enables reduced energy consumption and effectively mitigates safety hazards caused by certain flammable materials during production.

## Description

### TECHNICAL FIELD

The present invention relates to the field of antibacterial and perspiration-inhibiting cosmetics, and specifically, to an antibacterial and sun-protective powdered solid compact composition with decorative elements and a manufacturing method thereof.

### BACKGROUND

During high-temperature summer weather, skin exposed to intense sunlight is prone to sweating and developing unpleasant odors, and more severely, can easily lead to skin conditions such as miliaria and papules. It is known that various types of personal care cosmetics available on the market can, to varying degrees, inhibit sweat secretion, suppress the growth of resident bacteria, and provide protection against ultraviolet radiation damage.

For instance, a common type of solid powder product, which includes dusting powder in a loose form, can serve to inhibit perspiration and act as a bactericidal agent. It is typically applied by sprinkling or smearing onto the target area. However, its use is prone to generating airborne dust, which users (particularly infants, young children, and children) may inadvertently inhale, posing potential health risks. Consequently, industry counterparts have introduced solid powder compact products. By modifying the product formulation, powdered raw materials can be compressed into forms such as small round cakes or rounded rectangles. These can be applied to the skin using a puff or by direct application, forming a thin powder layer on the skin and thereby reducing dust dispersion.

Beyond the method of application, users also consider the functionality of such products from multiple perspectives. One key concern is their effectiveness in preventing skin dryness and itching. For example, some existing products incorporate antifungal agents containing heterocyclic structures, such as azole compounds. While these offer excellent bactericidal and bacteriostatic effects, thereby alleviating skin allergies, eczema, and other conditions, their use raises safety concerns. Such antimicrobial agents are known to possess animal teratogenic, carcinogenic potential, and cell mutagenic toxicity. Absorption through the skin epidermis into the plasma or bodily fluids may be detrimental to user health. Therefore, utilizing natural and standardized raw materials can more effectively safeguard consumer safety and interests.

Furthermore, in some solid powder products, inorganic porous powders such as calcium carbonate, magnesium carbonate, magnesium sulfate, and magnesium silicate are solely used as the primary powder materials. When applied to the skin, these products can excessively absorb moisture and release heat, leading to adverse reactions like dryness and itching. To address the functional goals of antibacterial and perspiration-inhibiting properties, some current products have incorporated aluminum starch octenylsuccinate, which adsorbs sweat and sebum, to partially replace inorganic porous powders. For example, the Chinese invention patent application CN115670972A utilizes aluminum starch octenylsuccinate as a material to mitigate the overly dry sensation during use. Additionally, during the intermediate processing stage of this technical solution, a powder compact form is achieved. However, as this solution involves adding a significant amount of high-concentration alcohol followed by high-temperature drying to evaporate the alcohol, the resulting compact structure tends to be porous and loose, exhibiting insufficient structural stability. Consequently, this solution adopts a loose powder form as the final product, which still unavoidably poses the risk of dust inhalation during use.

As another functional aspect, the level of sun protection is also a significant concern for users. The sun-protective effect is achieved by absorbing ultraviolet (UV) radiation. Some UV absorbers, such as octinoxate, are widely used in sunscreen cosmetics. However, for users with sensitive skin, these may cause adverse reactions like redness, itching, or allergies, potentially due to chemical changes or heat release during UV absorption. Moreover, increasing the amount of UV absorber often necessitates adding more oils, which can sometimes lead to an undesirable sticky feel and impair the user experience.

From a commercial promotion perspective, integrating products with specific, commercially significant IP characters can dramatically enhance consumer emotional trust and recognition. Products featuring highly recognizable IP images are more likely to achieve commercial success compared to basic products. Correspondingly, holders of established IP images, considering brand maintenance, impose relatively stringent quality inspection standards for products bearing their IP. However, existing solid compact products are limited by their ingredient selection; they can only be molded into relatively stable simple geometric shapes and still suffer from insufficient mechanical strength. For instance, the aforementioned solid compacts with simple surface patterns require use with a puff and cannot be directly held and applied to the skin. Another type of small, round solid compact can be held and applied directly, but due to its formulation constraints, incorporating decorative elements such as surface patterns, text, embossed shapes, angles, rounded corners, or chamfers often leads to instability, such as cracking or detachment at the junctions between the decorative elements and the main body. Therefore, from the perspective of image development and expansion, the commercial value of these existing products is relatively limited.

### SUMMARY

An object of the present invention is to provide an antibacterial and sun-protective powdered solid compact composition with decorative elements. Said composition exhibits excellent antibacterial and sun-protection efficacy, and the resulting product form can incorporate multiple stably shaped decorative elements, thereby enabling the product to express a variety of distinctive shapes. It demonstrates good structural stability during direct holding and application to the skin, while significantly reducing potential safety hazards associated with dust dispersion.

To achieve the aforementioned objective, the present invention adopts the following technical means:
An antibacterial and sun-protective powdered solid compact composition with decorative elements, consisting of the following raw materials in percentage by weight:
Aluminum Starch Octenylsuccinate and/or Calcium Starch Octenylsuccinate: 15%-25%
Carrier Powder: 45%-65%
Ultraviolet Protective Agent: 1%-10%
Antibacterial Agent: 0.1%-0.5%
Non-expandable Polymer Powder: 1%-5%
Viscous Fluid Skin-Feel Modifier: 5%-15%
Plant Extract: 0.1%-7.5%.

By refining the particle size of the solid raw material components among the aforementioned ingredients to 5-50 µm, and subjecting all raw materials to at least a thorough mixing step followed by compression molding within a mold, a powdered solid compact composition with a density of 8-9 g/cm³ is formed. This formulation retains the ultraviolet protective agent, viscous fluid skin-feel modifier, and antibacterial agent as functional components, ensuring users obtain the desired antibacterial and sun-protective efficacy during use. Preferably, the composition is formed with a density of 8.25-8.5 g/cm³. This not only results in a product with a structurally stable form but also enables smoother spreading of the powder on the skin during application.

More preferably, the particle size of the solid raw materials is 10-30 µm. Utilizing solid raw materials within this particle size range prevents excessive aggregation between the solid materials and the viscous fluid skin-feel modifier during the mixing process, thereby promoting better dispersion of the raw materials. Furthermore, it eliminates the coarse or gritty sensation that can occur upon application to the skin surface due to overly large particles.

Regarding the selection of the viscous fluid skin-feel modifier, it specifically refers to materials possessing a certain viscosity that can be adsorbed by the aluminum starch octenylsuccinate and/or calcium starch octenylsuccinate, as well as the carrier powder. Specific examples include silicone-based agents such as polymethylsiloxane, polyether siloxane, alkyl siloxane, polyalkylaryl siloxane, or polyether siloxane copolymers; or ester oils such as glyceryl monoricinoleate, isobutyl palmitate, glyceryl monostearate, isocetyl stearate, tallow sulfate, caprylic triglyceride, capric triglyceride, ethylhexyl palmitate, glyceryl tri(2-ethylhexanoate), diisobutyl adipate, isopropyl palmitate, 2-ethylhexyl palmitate, cetyl 2-ethylhexanoate, tocopheryl acetate; or alcoholic materials such as propylene glycol, triethylene glycol, butylene glycol, pentylene glycol, caprylyl glycol, 3-[2-(ethylhexyl)oxy]-1,2-propanediol, stearyl alcohol, oleyl alcohol, cetyl alcohol. Preferably, based on the total weight percentage, it is more suitable to formulate the viscous fluid skin-feel modifier to consist of 1%-2% silicone conditioning agent, 1%-5% synthetic ester oil, and 1%-11% alcohol. To accommodate users with varying skin sensitivities, low-irritancy viscous fluid skin-feel modifiers should be rationally selected. In light of this, and to ensure that a portion of the viscous fluid skin-feel modifier components can be adsorbed by the aluminum starch octenylsuccinate, calcium starch octenylsuccinate, and carrier powder, thereby maintaining a favorable structural form in the final compressed powdered solid compact composition, it is preferable that the silicone conditioning agent is selected from one or a mixture of polymethylsiloxane, polydimethylsiloxane, alkyl methyl siloxane, and polydimethylcyclosiloxane; the synthetic ester oil is selected from one or a mixture of caprylic triglyceride, capric triglyceride, ethylhexyl palmitate, glyceryl tri(2-ethylhexanoate), diisobutyl adipate, isopropyl palmitate, cetyl 2-ethylhexanoate, bis-diglyceryl polyacyladipate-2, and tocopheryl acetate; and the alcohol is selected from one or a mixture of propylene glycol, caprylyl glycol, butylene glycol, pentylene glycol, and 3-[2-(ethylhexyl)oxy]-1,2-propanediol. This combination can provide suitable fluidity and dispersibility to the powder particles upon application to the skin, effectively enhancing a favorable, micro-moist tactile response characterized by skin lubricity.

Furthermore, the ultraviolet protective agent is composed of one or a mixture of zinc oxide, cerium oxide, and titanium oxide. When using such ultraviolet protective agents, their addition amount can be reasonably increased to 5%-10% by total weight percentage. Compared to using lower proportions of ultraviolet protective agents with certain skin irritancy, such as avobenzone, octocrylene, oxybenzone, homosalate, octisalate, and octinoxate, the formulation of the present invention can employ a relatively higher proportion of metal oxide-based ultraviolet protective agents as raw materials. By appropriately using micronized metal oxides with suitable fineness, these micronized metal oxides can react with the viscous fluid skin-feel modifier and fatty acids in sebum, leading to their immobilization and suppressing the diffusion of sebum. This is conducive to the retention of the applied powder layer on the skin and provides a smooth tactile texture.

The choice of carrier powder can include other suitable, low-swelling particulate powder materials, such as mica, kaolin, silica, white clay, bentonite, magnesium stearate, aluminum magnesium silicate, and the like. Preferably, by total weight percentage, the carrier powder is composed of a mixture of 15%-20% mica, 10%-15% kaolin, 15%-20% magnesium stearate, and 5%-10% silica. The rational selection of the type and ratio of the carrier powder, in combination with aluminum starch octenylsuccinate and/or calcium starch octenylsuccinate, enables the adsorption of a portion of the viscous fluid skin-feel modifier. During the step of uniformly mixing the raw materials, this facilitates the formation of appropriately sized aggregated particles and allows the final product to achieve a balance between skin lubricity and a micro-moist tactile response, avoiding a dry powder feel or an undesirably heavy, sticky adherence.

The non-expandable polymer powder is uniformly mixed with the carrier powder, aluminum starch octenylsuccinate and/or calcium starch octenylsuccinate. When the viscous fluid skin-feel modifier is blended and dispersed with the aforementioned materials to form a loose powder material, each raw material exhibits different adsorption capacities for the modifier. Upon compression, this mixture enables the formation of a stable structure, while during application, the powder components can smoothly spread into a thin layer on the skin. Preferably, the non-expandable polymer powder is composed of one or a mixture of polyethylene powder, polytetrafluoroethylene powder, polyamide powder, and PMMA powder.

Furthermore, the plant extract is composed of one or a mixture of aloe vera extract, calendula extract, *Bambusa arundinacea* extract, sophora flavescens extract, salvia miltiorrhiza powder, ginger extract, and white willow bark extract.

The reasonable incorporation of plant extracts is crucial. When utilizing antibacterial agents at lower concentrations or those with non-potent efficacy, it is challenging for the product to achieve relatively ideal antibacterial pharmacokinetic effects against various resident bacteria. The combination of appropriately selected plant extracts possessing bactericidal and anti-inflammatory properties with the antibacterial agent can yield a potent antibacterial effect. Furthermore, the inventors have discovered that, beyond the perceived efficacy in inhibiting bacteria, the plant extracts can also provide certain anti-inflammatory, bactericidal, and anti-itching actions. In the application of the present invention as a body product for absorbing sweat and inhibiting bacteria, contact between the product and the hands, feet, neck, or other body parts, especially skin folds, can effectively reduce the incidence of adverse skin conditions such as miliaria and papules.

Based on the antibacterial and sun-protective powdered solid compact composition prepared according to the present invention, the decorative elements preferably have a minimum dimension of not less than 0.1 mm and a maximum dimension of not more than 25 mm. The ratio of the minimum dimension to the maximum dimension preferably ranges from 1:1 to 1:40. Appropriately, the overall volume should be suitable for convenient handling by hand, allowing users to easily hold and apply it to the skin surface. Given the rationally designed decorative elements described above, the resulting product can be formed into a variety of shapes, such as animals, overall trademark logos, or patterns with complex and highly detailed surface textures. This endows the product manufactured according to the present invention with significant commercial value.

The present invention also discloses a preparation method for the antibacterial and sun-protective powdered solid compact composition with decorative elements, which is produced through the following steps:
(1) Pre-mixing: At room temperature, add all solid raw materials, except the plant extract and the viscous fluid skin-feel modifier, into a powder mixing vessel according to their weight percentages. Stir at 2000-2500 rpm until uniformly dispersed, while controlling the temperature of the raw material mixture during stirring to not exceed 40°C.
(2) Mixing: Pre-disperse the plant extract into the viscous fluid skin-feel modifier raw material, then spray it into the powder mixing vessel using an atomization device. During the spraying process, intermittently stir multiple times at 2000-2500 rpm until all components are uniformly dispersed, while controlling the temperature of the raw material mixture during stirring to not exceed 40°C.
(3) Secondary Pulverization: Subject the thoroughly dispersed raw material mixture to pulverization using a high-speed pulverizer to convert it into a powder, and sieve the mixed powder through a 100-200 mesh screen.
(4) Standing: Allow the sieved mixed powder to stand thoroughly under environmental conditions of 25-28°C temperature and 40%-60% humidity until the temperature and humidity of the mixed powder stabilize.
(5) Compaction: Load the stood mixed powder into a mold, and compact the powder within the mold to form the powdered solid compact composition with a density of 8-9 g/cm³.

Compared to existing technical solutions, the present invention does not employ high-temperature processing of the product and eliminates the need to use flammable alcohol as a medium. This effectively removes unnecessary safety hazards during the manufacturing process. Furthermore, the resulting powdered solid compact composition differs from the porous, loose structure formed by high-temperature-induced alcohol evaporation; instead, its compressed structure exhibits excellent structural stability. Moreover, to further enhance the structural stability of the product and the production yield, the temperature of the raw material mixture during stirring in steps 1 and 2 is controlled not to exceed 40°C. This prevents excessively high temperatures from causing agglomeration between the aluminum/calcium starch octenylsuccinate and other raw materials, thereby ensuring a more uniform mixing of all components. Additionally, by adopting a processing method involving intermittent multiple compactions of the mixed, sieved powder at a pressure of 70-80 kg/cm², it is possible to ensure thorough degassing of the powdered compact composition within the mold. This effectively promotes uniform density throughout all parts of the composition, consequently reducing potential defects such as hollow cavities or delamination in the final product.

The present invention exhibits at least the following technical effects:
The powdered solid compact composition according to the present invention, when applied in a thin layer, forms a fine powder layer on the skin. This layer effectively absorbs moisture from the skin surface, helps refine the appearance of pores, and imparts a dry, film-like, smooth tactile sensation. The compact can be applied directly by holding it against the skin. During use, the solid powder structure remains stable, withstanding over 200 applications, and significantly reduces the concentration of inhalable powder dispersed into the air. This effectively mitigates the health risks associated with accidental powder inhalation.

The invention incorporates plant extracts, an ultraviolet protective agent, and a skin-feel modifier as functional components for antibacterial and sun-protection efficacy. After application, it effectively inhibits the persistence of resident bacteria on the applied area and reduces unpleasant odors caused by the bacterial decomposition of sweat and sebum. Furthermore, it provides a sun protection effect reaching a level of at least SPF 15 PA+.

The composition can be compressed into a solid form featuring multiple decorative elements, offering the potential for developing a variety of product appearances. This form is structurally stable, providing good portability and durability.

### BRIEF DESCRIPTION OF DRAWINGS

Figure 1 is a schematic diagram illustrating one potential product development form of the present invention.
Figures 2A to 2C are schematic diagrams comparing the application effects of the present invention, a commercially available dusting powder, and a commercially available solid powder compact, respectively.
Figures 3A to 3C are schematic diagrams comparing the application effects of the present invention, a commercially available dusting powder, and a commercially available solid powder compact, respectively.

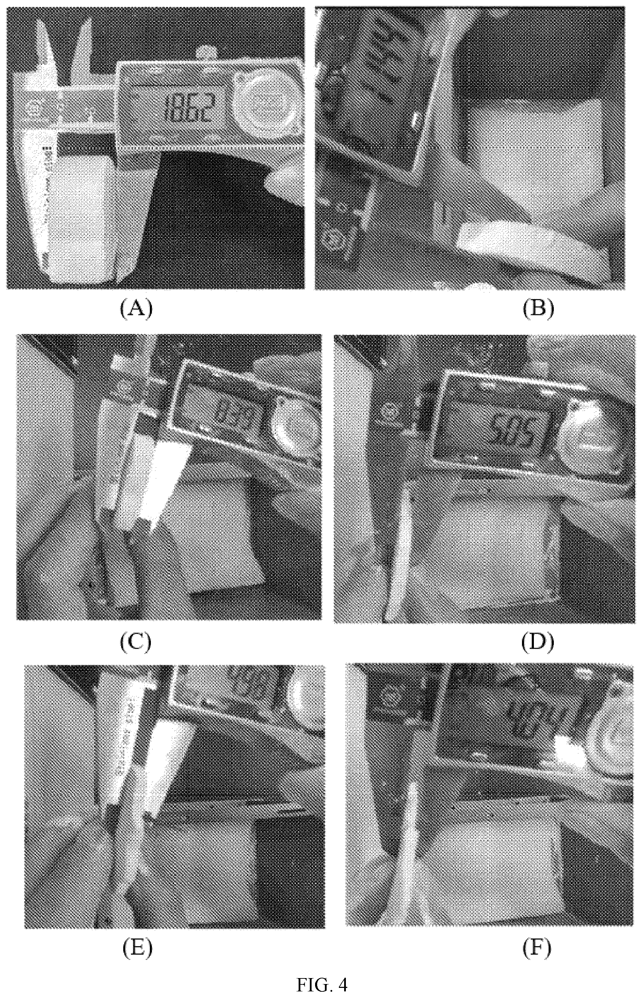

Figures 4A to 4F are schematic diagrams showing the state of the invention after 0 to 250 application cycles.
Figures 5A to 5C are cross-sectional electron micrograph morphology diagrams of Example 1 according to the present invention.

### DETAILED DESCRIPTION OF THE EMBODIMENTS

The embodiments of the present invention will be described in detail below. However, the present invention is not limited to the following embodiments. The preparation methods for Examples 1-8 described below were all conducted under ambient environmental conditions of approximately 25°C temperature and about 50% humidity, and identical production and processing equipment was used.

### Examples 1-4

The raw materials were formulated according to the following weight percentages:

| compo nent | Name of Raw Material | Example 1 | Example 2 | Example 3 | Example 4 |
|---|---|---|---|---|---|
| A | Aluminum Starch Octenylsuccinate | 20 | 18 | 25 | 17 |
| A | Mica | 16.5 | 20 | 19 | 19 |
| A | Kaolin | 13.5 | 12 | 11.7 | 14 |
| A | Magnesium Stearate | 17 | 16 | 16 | 18 |
| A | Silica | 8 | 6 | 10 | 7 |
| A | Polyethylene | 4 | 3 | 2 | - |
| A | PMMA | - | 2 | 2 | - |
| A | Polytetrachloroethylene | - | - | - | 1 |
| A | Zinc Oxide | 7 | 5.87 | 3 | 8 |
| A | Sodium Benzoate | 0.45 | 0.25 | 0.5 | 0.1 |
| B | Caprylic Triglyceride | 1.14 | 1.5 | 2 | 1.55 |
| B | Polydimethylsiloxane | 1.14 | 1 | - | 1.25 |
| B | Ethylhexyl Palmitate | 1.14 | 1.5 | 2 | 1.05 |
| B | Caprylyl Glycol | 0.1 | - | - | 0.2 |
| B | Ethylhexyl Glycine | - | 0.5 | | |
| B | Polydimethylsiloxane | 0.43 | - | 0.5 | 0.25 |
| B | Bis-diglyceryl Polyacyladipate-2 | 0.4 | 0.38 | 0.3 | 0.25 |
| C | Bambusa Arundinacea Stem Extract | 3 | 5 | 4.5 | 3 |
| C | Aloe Vera Extract - Butylene Glycol Mixture | 5 | 4 | 0.5 | 3 |
| C | Calendula Officinalis Flower Extract- Butylene Glycol Mixture | 1.2 | 3 | 1 | 5 |
| C | Sophora Flavescens Root Extract | - | - | - | 0.35 |

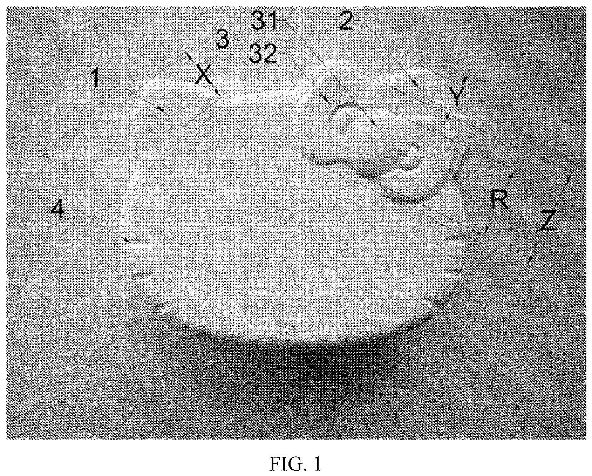

The raw materials for Examples 1-4 above were grouped into A, B, and C components. The products for Examples 1-4 were prepared by processing components A, B, and C separately according to the following steps:
(1) Pre-mixing: At ambient temperature, Component A-comprising all solid raw materials except the plant extract and the viscous fluid skin-feel modifier-was added to a powder mixing vessel according to its weight percentage. The mixture was stirred at 2000 rpm until uniformly dispersed, while controlling the temperature of the raw material mixture during stirring not to exceed 40°C.
(2) Mixing: The corresponding plant extract was pre-dispersed in butylene glycol at a 1:5 ratio. Subsequently, the mixed Component B and the mixed Component C were sequentially sprayed into the powder mixing vessel in 6 separate batches each using an atomization device, according to their weight percentages. During the spraying process, intermittent stirring was performed at 2500 rpm for 60 seconds each time, until all components were uniformly dispersed, while controlling the temperature of the raw material mixture during stirring not to exceed 40°C.
(3) Secondary Pulverization: The thoroughly dispersed raw material mixture was pulverized using a high-speed pulverizer. Pulverization was performed twice until the mixture attained a powdered form. After each pulverization, the powder was sieved through a 100-mesh screen, resulting in the mixed sieved powder.
(4) Standing: The mixed sieved powder was allowed to stand thoroughly under environmental conditions of 25-28°C temperature and 40%-60% humidity, until the temperature and humidity of the powder stabilized.
(5) Compaction: The mixed sieved powder, after standing, was loaded into a mold. The powder within the mold was compacted at a pressure of 75 kg/cm². Compaction was performed 4 times, with a dwell time of (3) Seconds for each compression. After demolding, the products obtained for Examples 1-4 all exhibited the antibacterial and sun-protective powdered solid compact composition with decorative elements, as shown in Figure 1, with a density ranging from 8.25 to 8.45 g/cm³.

### Examples 5-8

The raw materials were formulated according to the following weight percentages:

| compo nent | Name of Raw Material | Example 5 | Example 6 | Example 7 | Example 8 |
|---|---|---|---|---|---|
| A | Calcium Starch Octenylsuccinate | 7 | 10 | 17 | 3.55 |
| A | Aluminum Starch Octenylsuccinate | 11 | 10 | 6 | 15 |
| *A* | Mica | 18 | 15 | 17.5 | 19 |
| A | White Clay | 14 | 15.2 | 15 | 17.35 |
| A | Magnesium Stearate | 17 | 17 | 15 | 18 |
| A | Silica | 7 | 7 | 8.5 | 7 |
| A | Polyethylene | 2 | 2.5 | . | 0.5 |
| A | Polyamide | 2.1 | 2.5 | 4 | 0.5 |
| A | Titanium Oxide | 5.87 | 5 | 7 | 8 |
| A | Zinc Oxide | 1 | 2 | - | - |
| A | Sodium Sorbate | 0.15 | 0.45 | 0.3 | 0.2 |
| B | Glyceryl Monostearate | 1.5 | 1.1 | 2 | 1.25 |
| B | Polyether Siloxane | 0.5 | 1.2 | 0.5 | 1.5 |
| B | Isocetyl Stearate | 2 | 1.4 | 0.4 | 1.1 |
| B | Caprylyl Glycol | - | 0.1 | - | 0.2 |
| B | Ethylhexyl Glycine | 0.5 | - | - | 0.25 |
| B | 2-Ethylhexyl Palmitate | - | 0.4 | 0.5 | - |
| B | Bis-diglyceryl Polyacyladipate-2 | 0.38 | - | 0.3 | 0.25 |
| C | Salix Alba Bark Extract | 4 | 3 | 2.5 | 3 |
| C | Ginger Extract- Propylene Glycol Mixture | 5 | 4 | 2.5 | 3 |
| C | Calendula Officinalis Flower Extract- Pentylene Glycol Mixture | 1 | 2.15 | 1 | 0.35 |

The raw materials for Examples 5-8 above were grouped into A, B, and C components. The products for Examples 5-8 were prepared by processing components A, B, and C separately according to the following steps:
(1) Pre-mixing: At ambient temperature, Component A-comprising all solid raw materials except the plant extract and the viscous fluid skin-feel modifier-was added to a powder mixing vessel according to its weight percentage. The mixture was stirred at 2500 rpm until uniformly dispersed, while controlling the temperature of the raw material mixture during stirring not to exceed 40°C.
(2) Mixing: Ginger extract was pre-dispersed in propylene glycol at a 1:5 ratio, and calendula extract was pre-dispersed in pentylene glycol at a 1:4 ratio. Subsequently, the mixed Component B and the mixed Component C were sequentially sprayed into the powder mixing vessel in 6 separate batches each using an atomization device, according to their weight percentages. During the spraying process, intermittent stirring was performed at 2500 rpm for 60 seconds each time, until all components were uniformly dispersed, while controlling the temperature of the raw material mixture during stirring not to exceed 40°C.
(3) Secondary Pulverization: The thoroughly dispersed raw material mixture was pulverized using a high-speed pulverizer. Pulverization was performed twice until the mixture attained a powdered form. After each pulverization, the powder was sieved through a 200-mesh screen, resulting in the mixed sieved powder.
(4) Standing: The mixed sieved powder was allowed to stand thoroughly under environmental conditions of 25-28°C temperature and 40%-60% humidity, until the temperature and humidity of the powder stabilized.
(5) Compaction: The mixed sieved powder, after standing, was loaded into a mold. The powder within the mold was compacted at a pressure of 80 kg/cm². Compaction was performed 4 times, with a dwell time of (3) Seconds for each compression. After demolding, the products obtained for Examples 1-4 all exhibited the antibacterial and sun-protective powdered solid compact composition with decorative elements, as shown in Figure 1, with a density ranging from 8.5 to 8.75 g/cm³.

### Product Form Evaluation

Examples 1-8 were all compacted using the same mold to achieve the form shown in Figure 1. Structurally, the product overall presents the shape of a cat's head, featuring various decorative elements at multiple locations such as the left ear 1, right ear 2, bow 3, and whiskers 4. The specific parameters for each decorative part are as follows:
The heights of both the left ear 1 and the right ear 2 are 18.62 mm. The distance X from the base to the tip of the left ear 1 is 10 mm. The distance Y from the base to the tip of the right ear 2 is 6 mm. Thus, the ratio of the minimum dimension to the maximum dimension for the left ear 1 is approximately 1:1.86. The ratio for the right ear 2 is approximately 1:3.1.

The highest point of the bow center 31 has a height of 0.8 mm relative to the main body. The longest chord length R of the bow center 31 is 10 mm. Thus, the ratio of the minimum dimension to the maximum dimension for the bow center 31 is 1:12.5. The highest point of the bow side petal 32 has a height of 0.5 mm relative to the main body. The longest dimension Z of the bow side petal 32 is 15 mm. Thus, the ratio of the minimum dimension to the maximum dimension for the bow side petal 32 is 1:30.

The whiskers 4 have a depth of 0.(2) Mm relative to the main body. The length of the whiskers 4 is 4 mm. Thus, the ratio of the minimum dimension to the maximum dimension for the whiskers 4 is 1:20.

The products from Examples 1-8, manufactured using their respective processing methods, all successfully formed structures that were robust with clear, well-defined textures. These compositions exhibited high structural stability, allowing them to be held directly for application without chipping or breaking. Upon application to the skin, the powder layer imparted a dry, film-like, smooth tactile sensation. Furthermore, the products demonstrated both antibacterial and sun-protective effects. The structural stability and efficacy of the products were verified through the following tests and observations: hardness testing, application durability testing, dust impact testing, application effect testing, antibacterial efficacy testing, sun protection efficacy testing, and cross-sectional electron microscopy. The test results are summarized below:

### Hardness Testing

This evaluation employed three testing methods-horizontal translation drop test, air column bag drop test, and push-pull gauge hardness test-to determine whether the product hardness of Examples 1-8 met specifications.

Horizontal Translation Drop Test: Test samples from Examples 1-8 were placed on a shelf 100 cm high. They were pushed horizontally off the edge to fall freely onto the ground twice, simulating accidental drops during user handling. The structural stability of the products was observed. The test results indicated that only the product from Example 7 exhibited localized damage (to the tip of the left ear decorative element) after the second drop. The structures of all other products remained stable, resulting in a passing test outcome for them.

Air Column Bag Drop Test: A single product, housed within its packaging box containing a plastic blister tray insert, was wrapped in an air column cushioning bag. The tester threw the package a distance of 4-5 meters onto the ground, simulating extreme handling conditions during shipping. This was repeated consecutively five times. Testing confirmed that all products from Examples 1-8 maintained structural integrity.

Push-Pull Gauge Hardness Test: An HANDPI NK-500 dial push-pull gauge, fitted with a 1 cm² flat test head, was used as the hardness testing apparatus. Hardness testing was performed on products from Examples 1-8 by pressing the flat test head against the test area until fracture occurred, with the recorded value noted. The test results are as follows:

| Position Hardness | Example 1 | Example 2 | Example 3 | Example 4 | Example 5 | Example 6 | Example 7 | Example 8 |
|---|---|---|---|---|---|---|---|---|
| Body Hardness (kg/cm²) | 9.4 | 9.5 | 9.8 | 9.3 | 10.1 | 9.2 | 9.1 | 9.8 |
| Left Ear Hardness (kg/cm²) | 4.9 | 5.0 | 5.1 | 5.0 | 5.2 | 5.0 | 5.0 | 5.1 |
| Right Ear Hardness ( kg/cm²) | 5.0 | 5.1 | 5.3 | 5.1 | 5.2 | 5.0 | 5.0 | 5.3 |

The test results demonstrate that the hardness values for Examples 1-8 are relatively similar and are sufficient to withstand commercial transportation and routine user handling.

### Application Effect Testing

Products from Example 1, a commercially available dusting powder, and a commercially available solid powder compact were used as test subjects. Their performance during application was evaluated using two methods: application on natural skin and application on skin in a perspired state.

### Application on Natural Skin Test:

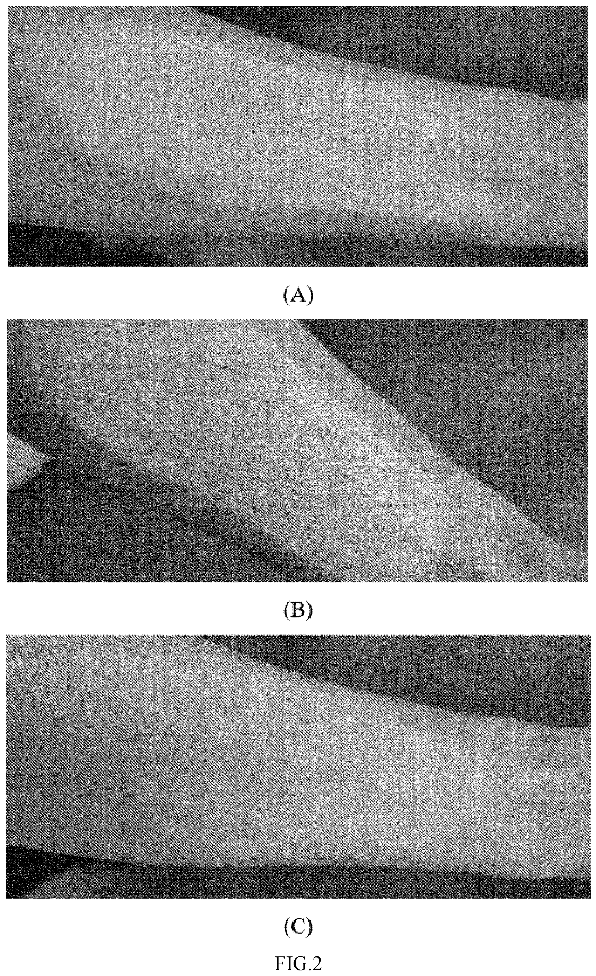

Figure 2A shows the state after application of the Example 1 product onto naturally dry skin surface. Figure 2B shows the state after application of the commercially available dusting powder onto naturally dry skin surface. Figure 2C shows the state after application of the commercially available solid powder compact onto naturally dry skin surface.

Regarding Example 1 product, upon application to the skin surface, it formed a fine, thin powder layer. The thickness of the powder layer was uniform across different areas, and the overall colour consistency was strong.

Regarding commercially available dusting powder, upon application to the skin surface, it formed a relatively coarse, thin powder layer. A significant amount of loose powder was present on the skin, which could easily transfer to clothing and was inconvenient to manage during cleanup.

Regarding commercially available solid powder compact, upon application to the skin surface, it formed a fine, thin powder layer. However, the initial application area showed the presence of loose powder, requiring the user to perform a secondary application to achieve uniformity.

### Application on Perspired Skin Test:

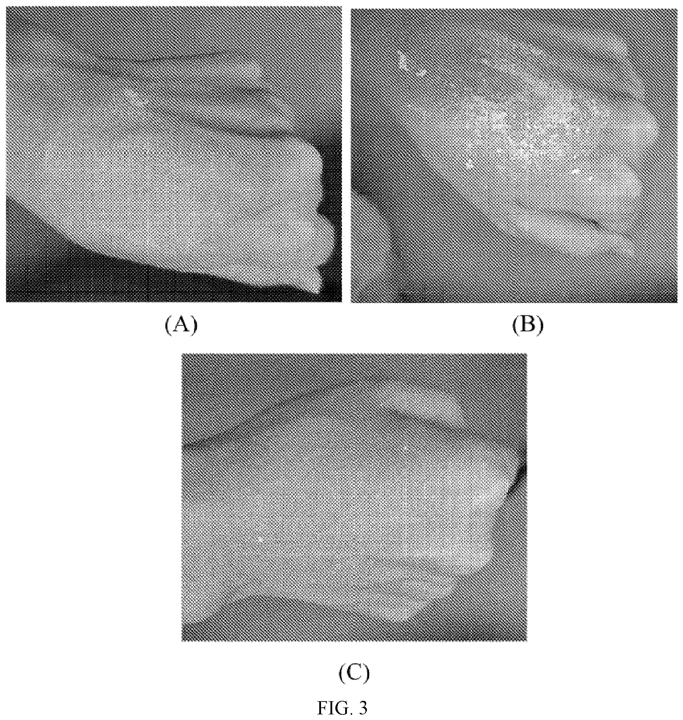

Figure 3A shows the state after application of the Example 1 product onto a skin surface that had been wiped dry but retained a sticky, damp feeling. Figure 3B shows the state after application of the commercially available dusting powder onto the same type of skin surface. Figure 3C shows the state after application of the commercially available solid powder compact onto the same type of skin surface.

Regarding the product in Figure 3A, upon application to the skin surface, it formed a fine, thin powder layer. It effectively absorbed moisture from the skin surface and helped refine the appearance of pores. Users experienced a dry, film-like, smooth tactile sensation.

Regarding the product in Figure 3B, upon application to the skin surface, obvious clumping and aggregation of the powder occurred in multiple areas, resulting in a coarse texture. Users needed to remove the aggregated powder clumps before attempting a secondary application. Additionally, users perceived a slight warming sensation and dryness during use.

Regarding the product in Figure 3C, upon application to the skin surface, the powder layer appeared dissolved and indistinct, offering relatively weak moisture-absorbing performance.

### Application Durability Test

The product from Example 2 was used as the test sample. It was applied to a paper substrate with each application stroke being approximately 10-15 cm in length. The relationship between the number of application strokes and the reduction in product thickness was observed. The test continued until the main body or decorative elements of the product exhibited structural failure. The test results are as follows:

| Product Thickness Measured Photo | Simulated Application Number | Powder Thickness | Cracking or Not |
|---|---|---|---|
| Figure 4A | 0 | 18.62MM | Integrity without Cracking |
| Figure 4B | 100 | 11.44 MM | Integrity without Cracking |
| Figure 4C | 150 | 8.39 MM | Integrity without Cracking |
| Figure 4D | 200 | 5.05 MM | Integrity without Cracking |
| Figure 4E | 220 | 4.98 MM | Slightly Cracking |
| Figure 4F | 250 | 4.04 MM | Cracking into Two |

The results indicate that the product can be applied by holding it directly, and it exhibits high durability during use.

### Dust Impact Effect Test

A PC-3A laser inhalable dust continuous tester (manufactured by Qingdao Jingcheng Instrument Co., Ltd.) was used as the testing instrument. Measurements were taken of the initial ambient air, followed by the air during the use of a commercially available loose powder dusting powder, a commercially available solid powder compact (applied with a puff), and the products prepared according to Examples 1, 3, and 8. The test simulated natural application by a user. After 30 seconds of natural application, the changes in the concentrations of PM2.5 and PM10 in the air were measured within a test range 50 cm from the user. The test results are as follows:

| Test Item | Initial Environment State | Commercial Dusting Powder | Commercial Solid Powder Compact | Example 1 | Example 3 | Example 8 |
|---|---|---|---|---|---|---|
| pm2.5 (mg/m³) | 0.009 | 0.026 | 0.020 | 0.010 | 0.010 | 0.010 |
| µm10 (mg/m³) | 0.038 | 0.560 | 0.512 | 0.047 | 0.048 | 0.043 |

The measurements demonstrate that compared to the other products, the use of Examples 1, 3, and 8 resulted in a significant reduction in the concentration of inhalable particles within the tested spatial range. Furthermore, the concentration of fine particulate matter within this range during use of these examples was lower. This effectively reduces the health risk associated with user inhalation of dust during product application.

### Antibacterial Efficacy Test

The product prepared according to Example 4 was tested for its bactericidal effect using the Carrier Immersion Bactericidal Test method outlined in the "Technical Standard for Disinfection". The test microorganisms selected were three common resident bacteria: *Staphylococcus aureus, Pseudomonas aeruginosa, and Escherichia coli.* The antibacterial efficacy of the product was determined, and the test results are as follows:

| Action Duration | Test Bacteria | Positive Control Colony Count (CPU/g) | Product Bacterial Colony Count (CFU/g) | Kill Log Value | Kill Rate (%) |
|---|---|---|---|---|---|
| Original Sample 2 mins | Staphylococcus aureus | 5.8×10⁴ | 1.1×10⁴ | 4.72 | 99.9981% |
| | Pseudomonas aeruginosa | 6.7×10⁴ | 1.2×10⁴ | 4.75 | 99.9982% |
| | Escherichia coli | 7.3×10⁴ | 1.1×10⁴ | 4.82 | 99.9985% |
| Original Sample 5 mins | Staphylococcus aureus | 5.8×10⁴ | 9.9×10³ | 4.77 | 99.9983% |
| | Pseudomonas aeruginosa | 6.7×10⁴ | 1.1×10⁴ | 4.78 | 99.9984% |
| | Escherichia coli | 7.3×10⁴ | 1.1×10⁴ | 4.82 | 99.9985% |
| Original Sample 10 mins | Staphylococcus aureus | 5.8×10⁴ | 7.5×10³ | 4.89 | 99.9987% |
| | Pseudomonas aeruginosa | 6.7×10⁴ | 8.0×10³ | 4.92 | 99.9988% |
| | Escherichia coli | 7.3×10⁴ | 9.4×10³ | 4.89 | 99.9987% |

The test confirmed that the product exhibits strong bactericidal action against *Staphylococcus aureus, Pseudomonas aeruginosa,* and *Escherichia coli,* achieving a kill rate of over 99.99% for each.

### Sun Protection Effect Test

The products prepared according to Examples 1, 3, 5, and 7 were tested using the Sun Protection Factor (SPF) Test Method specified in the "Cosmetics Safety Technical Specifications". The test instrument used was a Sun Protection Factor Analyzer, model SPF-290AS. The test environment was maintained at 21±1°C and 50±10% relative humidity.

### Test Procedure:

(1) Secure 3M tape, artificial skin, or a PVC film onto the sample plate.
(2) First, measure the baseline curve of the blank substrate. Then, apply the sample uniformly onto the 3M tape using dots. Using a finger covered with a latex glove, spread the sample gently and evenly over a 50 cm² area of the 3M tape at an application density of (2) Mg/cm².
(3) Place the sample plate after application in a dark environment at a temperature of 20±2°C and relative humidity of 50±5% to dry naturally for 20 minutes.
(4) Turn on the instrument power and preheat for 30 minutes. Once confirmed to be operating normally, use the SPF-290AS analyzer to select 6 points on the 3M tape. Measure and plot the Monochromatic Protection Factor (MPF) curves for these 6 wavelengths. The accompanying software then performs calculation and analysis, outputting the SPF value and standard deviation for the single test, as well as the SPF value for each scanning point. The test results are as follows:

| Sample | Label Value | SPF Measured Value | UVA PF Measured Value | Adjacent Wavelength Measured Value | Sum Protection Rating |
|---|---|---|---|---|---|
| Example 1 | N/A | 20 | 19.5 | 372.60 | SPF15 PA+ |
| Example 3 | N/A | 21 | 30.5 | 376.20 | SPF15 PA++ |
| Example 5 | N/A | 21 | 20 | 376.10 | SPF15 PA++ |
| Example 7 | N/A | 21 | 20 | 376.10 | SPF15 PA++ |

The results above indicate that the products from Examples 1, 3, 5, and 7 all achieved a sun protection rating of SPF 15 PA+ or higher, capable of meeting users' daily sun protection needs.

### Cross-sectional Electron Microscopy Observation

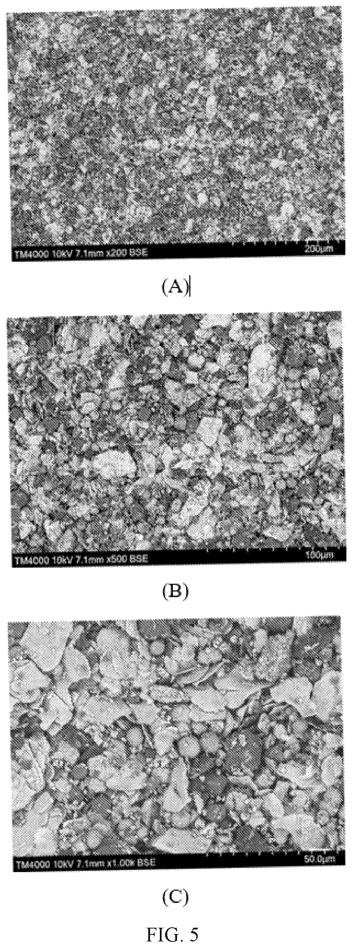

The product from Example 1 was used as the sample. Observation of the internal morphology and structure was conducted using a Hitachi benchtop scanning electron microscope (TM4000PlusII) under the following conditions: acceleration voltage of 10 kV, signal mode BSE, working distance of 7.1 mm, and direct observation without gold sputtering. Micrographs were taken at magnifications of x200, x500, and x1000, as shown in Figures 5A, 5B, and 5C, respectively.

The electron micrographs reveal that the aluminum starch octenylsuccinate, the low-swelling particulate powder, and the non-expandable polymer powder are well dispersed. Furthermore, the aluminum starch octenylsuccinate and the low-swelling particulate powder exhibit different absorption capacities for the viscous fluid skin-feel modifier, leading to the formation of particulate structures of varying sizes. Under compression, some of these structures undergo deformation into irregular curved, concave-convex, flake-like, or three-dimensional polygonal forms. Microscopically, adjacent particulate structures demonstrate mutual interlocking and embedding. Macroscopically, this translates into a product with high overall structural stability. During application to the skin, these fine particulate structures form a delicate, thin powder layer. The aluminum starch octenylsuccinate and the low-swelling particulate powder further absorb moisture from the skin, drying the skin surface and providing a micro-moist tactile response.

Finally, it should be noted that the above embodiments are merely illustrative of the technical solutions of the present invention and are not intended to limit the scope of protection. Although the present invention has been described in detail with reference to preferred embodiments, those skilled in the art will understand that modifications or equivalent replacements to the technical solutions of the present invention may be made without departing from the spirit and scope of the technical solutions of the present invention.

## Claims

1. An antibacterial and sun-protective powdered solid compact composition with decorative elements, **characterized by** comprising the following raw materials in percentage by weight:
Aluminum starch octenylsuccinate and/or calcium starch octenylsuccinate: 15%-25%;
Carrier powder: 45%-65%;
Ultraviolet protective agent: 1%-10%;
Antibacterial agent: 0.1%-0.5%;
Non-expandable polymer powder: 1%-5%;
Viscous fluid skin-feel modifier: 5%-15%;
Plant extract: 0.1%-7.5%;
wherein:
the carrier powder is one or a mixture of mica, kaolin, silica, white clay, bentonite, magnesium stearate, and aluminum magnesium silicate;
the non-expandable polymer powder is one or a mixture of polyethylene powder, polytetrafluoroethylene powder, polyamide powder, and PMMA powder;
the solid raw materials have a particle size of 5-50 µm, and the raw materials are thoroughly mixed and compressed to form a powdered solid compact composition with a density of 8-9 g/cm³.

2. The antibacterial and sun-protective powdered solid compact composition with decorative elements according to claim 1, **characterized in that**: the solid raw materials have a particle size of 10-30 µm.

3. The antibacterial and sun-protective powdered solid compact composition with decorative elements according to claim 1, **characterized in that**: based on the total weight percentage, the viscous fluid skin-feel modifier consists of 1%-2% silicone conditioning agent, 1%-5% synthetic ester oil, and 1%-11% alcohol.

4. The antibacterial and sun-protective powdered solid compact composition with decorative elements according to claim 3, **characterized in that**:
the silicone conditioning agent is selected from one or a mixture of polymethylsiloxane, polydimethylsiloxane, alkyl methyl siloxane, and polydimethylcyclosiloxane;
the synthetic ester oil is selected from one or a mixture of caprylic triglyceride, capric triglyceride, ethylhexyl palmitate, glyceryl tri(2-ethylhexanoate), diisobutyl adipate, isopropyl palmitate, cetyl 2-ethylhexanoate, and tocopheryl acetate;
the alcohol is selected from one or a mixture of propylene glycol, caprylyl glycol, butylene glycol, pentylene glycol, and 3-[2-(ethylhexyl)oxy]-1,2-propanediol.

5. The antibacterial and sun-protective powdered solid compact composition with decorative elements according to claim 1, **characterized in that**: based on the total weight percentage, the ultraviolet protective agent is one or a mixture of zinc oxide, cerium oxide, and titanium oxide, added in an amount of 5%-10%.

6. The antibacterial and sun-protective powdered solid compact composition with decorative elements according to claim 1, **characterized in that**: based on the total weight percentage, the carrier powder is composed of a mixture of 15%-20% mica, 10%-15% kaolin, 15%-20% magnesium stearate, and 5%-10% silica.

7. The antibacterial and sun-protective powdered solid compact composition with decorative elements according to claim 1, **characterized in that**: the plant extract is one or a mixture of aloe vera extract, calendula extract, *Bambusa arundinacea* extract, sophora flavescens extract, salvia miltiorrhiza powder, ginger extract, and white willow bark extract.

8. The antibacterial and sun-protective powdered solid compact composition with decorative elements according to any one of claims 1-7, **characterized in that**: the decorative elements have a minimum dimension of not less than 0.1 mm and a maximum dimension of not more than 25 mm, with a ratio of the minimum dimension to the maximum dimension ranging from 1:1 to 1:40.

9. A method for preparing the antibacterial and sun-protective powdered solid compact composition with decorative elements according to any one of claims 1-7, **characterized by** the following steps:
(1) Pre-mixing: At room temperature, add all solid raw materials, except the plant extract and the viscous fluid skin-feel modifier, into a powder mixing vessel according to their weight percentages; Stir at 2000-2500 rpm until uniformly dispersed, while controlling the temperature of the raw material mixture during stirring to not exceed 40°C;
(2) Mixing: Pre-disperse the plant extract into the viscous fluid skin-feel modifier raw material, then spray it into the powder mixing vessel using an atomization device; During the spraying process, intermittently stir multiple times at 2000-2500 rpm until all components are uniformly dispersed, while controlling the temperature of the raw material mixture during stirring to not exceed 40°C;
(3) Secondary Pulverization: Subject the thoroughly dispersed raw material mixture to pulverization using a high-speed pulverizer to convert it into a powder, and sieve the mixed powder through a 100-200 mesh screen;
(4) Standing: Allow the sieved mixed powder to stand thoroughly under environmental conditions of 25-28°C temperature and 40%-60% humidity until the temperature and humidity of the mixed powder stabilize;
(5) Compaction: Load the stood mixed powder into a mold, and compact the powder within the mold to form the powdered solid compact composition with a density of 8-9 g/cm³.

10. The method for preparing the antibacterial and sun-protective powdered solid compact composition with decorative elements according to claim 9, **characterized in that**: in step (5), the mixed sieved powder is compacted intermittently multiple times at a pressure of 70-80 kg/cm².
